# EUROPÄISCHE PATENTSCHRIFT

(11) **EP 3 057 558 B1**
(45) Veröffentlichungstag und Bekanntmachung des Hinweises auf die Patenterteilung: **28.03.2018**
(21) Anmeldenummer: 14796723.6
(22) Anmeldetag: 17.10.2014
(51) Int. Cl.: A61G 13/12

(54) **PATIENTENLAGEREINHEIT FÜR EINE VORRICHTUNG ZUM LAGERN EINES ZU RÖNTGENDEN PATIENTEN WÄHREND EINER OPERATION**
PATIENT-SUPPORTING UNIT FOR A DEVICE FOR SUPPORTING A PATIENT, WHO IS TO BE X-RAYED, DURING AN OPERATION
UNITÉ DE POSITIONNEMENT D'UN PATIENT POUR UN DISPOSITIF DESTINÉ À POSITIONNER UN PATIENT À RADIOGRAPHIER PENDANT UNE OPÉRATION

(30) Priorität: 18.10.2013 DE 102013111519
(43) Veröffentlichungstag der Anmeldung: 24.08.2016
(73) Patentinhaber: MAQUET GmbH, 76437 Rastatt (DE)
(72) Erfinder: KAISER, Jochen, 76185 Karlsruhe (DE); WYSLUCHA, Ulrich, 76356 Weingarten (DE); PETER, Stefan, 76437 Rastatt (DE); HUND, Siegfried, 77704 Oberkirch (DE)
(74) Vertreter: Zacco GmbH
(86) Internationale Anmeldenummer: PCT/EP2014/072324
(87) Internationale Veröffentlichungsnummer: WO 2015/055819

(56) Entgegenhaltungen:
- US-A- 5 147 287
- US-A- 5 806 117

## Beschreibung

Die Erfindung betrifft eine Patientenlagereinheit für eine Vorrichtung zur Auflage eines Körperteils eines zu röntgenden Patienten während einer Operation, die eine Befestigungseinheit zur Befestigung der Patientenlagereinheit an einer Schiene der Vorrichtung und ein Auflagepad zur Auflage des Patienten umfasst.

Bei verschiedenen Operationen, insbesondere bei Operationen an der Wirbelsäule, ist es notwendig, dass der Patient während der Operation geröntgt werden kann. Hierzu werden häufig C-förmige Röntgenapparate verwendet, wobei in der Öffnung des "C" der Patient gelagert ist. Insbesondere werden auch um bis zu 270° verschwenkbare C-Bögen zur Aufnahme von 3D-Bildern verwendet. Die Verwendung von klassischen Operationstischen zur Lagerung des Patienten ist nicht oder nur eingeschränkt möglich, da ein Röntgen nur in sehr beschränktem Umfang möglich ist. Zur Aufnahme von 3D-Aufnahmen muss der C-Bogen möglichst nah am Patienten über einen möglichst großen Bereich bewegt werden können. Die standardmäßigen Patientenauflagen von Operationstischen sind hierfür zu breit ausgebildet. Zum anderen umfasst der Operationstisch häufig dicke, metallhaltige Konstruktionen, so dass ein Röntgen nur in unzureichender Qualität möglich ist. Erschwerend kommt hinzu, dass die Dicke und Konturen der Konstruktionen sehr unterschiedlich sind, was die Qualität des Röntgenbildes weiter negativ beeinflusst und die Röntgenaufnahme über den C-Bogen limitiert.

Daher werden Vorrichtungen zum Lagern des Patienten verwendet, die an den Operationstisch angebaut werden können. Der Patient liegt dann mit dem Oberkörper, an dem operiert wird und der geröntgt werden muss, auf der angebauten Vorrichtung und lediglich mit seinen Beinen auf dem eigentlichen Operationstisch.

Eine solche Vorrichtung zum Lagern eines Patienten während einer Operation ist beispielsweise aus dem Dokument US 7,600,281 B2 bekannt. Die dort beschriebene Vorrichtung umfasst zwei parallel zueinander verlaufende Holme, die an der einen Seite am Operationstisch und an der anderen Seite an einem Ständer befestigt sind. An den Holmen sind mehrere Auflageflächen vorgesehen, auf denen der Patient, insbesondere dessen Oberkörper und dessen Hüften, gelagert werden können. Diese Auflageflächen ragen hierbei über den gesamten Bereich zwischen den beiden Holmen und verbinden diese.

Die zuvor beschriebene Vorrichtung hat den Nachteil, dass durch die Auflageelemente die Qualität eines aufgenommenen Röntgenbildes negativ beeinflusst werden kann. Die Konturen der Auflageelemente finden sich im Röntgenbild wieder, was ggf. zu Fehlinterpretationen führen kann. Darüber hinaus ermöglichen die starren Auflageelemente nur eine unzureichende Anpassung an die individuelle Anatomie des Patienten, so dass dieser ggf. nicht optimal für die Operation gelagert werden kann. Eine weitere Vorrichtung zum Lagern eines Patienten ist bekannt aus US 5,147,287. Es ist Aufgabe der Erfindung, eine Patientenlagereinheit für eine Vorrichtung zum Lagern eines zu röntgenden Patienten während einer Operation anzugeben, die eine optimale sichere Lagerung des Patienten ermöglicht.

Diese Aufgabe wird durch eine Patientenlagereinheit mit den Merkmalen des Anspruchs 1 gelöst. Vorteilhafte Weiterbildungen der Erfindung sind in den abhängigen Ansprüchen angegeben.

Erfindungsgemäß umfasst die Patientenlagereinheit eine Befestigungseinheit zur Befestigung der Patientenlagereinheit an einer Schiene der Vorrichtung zum Lagern des Patienten und ein Auflagepad zur Auflage eines Körperteils des Patienten. Ferner ist eine Höhenverstelleinheit zum Verändern des Abstandes zwischen der Befestigungseinheit und dem Auflagepad innerhalb eines vorbestimmten Einstellbereiches vorgesehen. Hierdurch wird erreicht, dass die Höhe des Auflagepads, auf dem ein Teilbereich des Körpers des Patienten aufliegt, relativ zur Befestigungseinheit und somit relativ zur Schiene, auf der die Patientenlagereinheit gelagert ist, eingestellt werden kann. Hiermit kann die Lagerung des Patienten optimal an die individuellen anatomischen Gegebenheiten des Patienten und die jeweilige durchzuführende Operation angepasst werden.

Im Gegensatz zum Stand der Technik kann auf das Stapeln von Polstern zur Erhöhung einzelner Körperbereiche verzichtet werden. Durch die Höhenverstellung des Auflagepads mit Hilfe der Höhenverstelleinheit ist das Auflagepad immer fest mit der Schiene verbunden, so dass dieses, anders als ein Polsterstapel, nicht verrutschen kann. Darüber hinaus wird durch ein solches Auflagepad, im Gegensatz zu Kissen, die Durchlässigkeit für Röntgenstrahlen nicht verändert. Bei dem Auftürmen von mehreren Polstern wird die Durchlässigkeit für Röntgenstrahlen umso schlechter, je mehr Kissen verwendet werden, so dass eventuell nur noch Röntgenbilder mit sehr schlechter Qualität aufgenommen werden können. Darüber hinaus birgt das Übereinanderstapeln mehrerer Polster die Gefahr, dass diese gegeneinander verrutschen und somit die Lagerung des Patienten nicht mehr sicher gegeben ist.

Das Auflagepad umfasst vorzugsweise eine feste Trägerstruktur und ein an dieser befestigtes Polster, so dass der Patient bequem abgestützt ist.

Die Patientenlagereinheit ist insbesondere derart ausgebildet, dass sie an Schienen einer Vorrichtung zum Lagern eines zu röntgenden Patienten angebracht werden kann. Um einen Patienten insbesondere während einer Wirbelsäulenoperation jederzeit röntgen zu können, wird dieser nicht auf dem eigentlichen Operationstisch, sondern auf einer Vorrichtung gelagert, die an diesem Operationstisch angebracht wird. Eine solche Vorrichtung umfasst zwei parallel zueinander verlaufende Schienen, von denen jeweils ein Ende an dem Operationstisch, insbesondere an den Schweißstellen des Operationstisches für Beinplatten, befestigt werden und die anderen Enden an einem zusätzlichen Ständer befestigt sind. An den Schienen werden dann mehrere dieser Patientenlagereinheiten angebracht, über die verschiedene Bereiche des Körpers des Patienten gelagert werden. Die einzelnen Patientenlagereinheiten sind auf den Schienen individuell verschiebbar und hierdurch, zusammen mit der Höhenverstellung, einfach individuell an die Anatomie des Patienten anpassbar. Insbesondere werden bei einer Wirbelsäulenoperation zwei solcher Patientenlagereinheiten zum Lagern des Oberkörpers und zwei weitere zum Lagern der Hüften des Patienten verwendet. Die Beine des Patienten liegen in diesem Fall insbesondere auf dem Mittelteil und/oder der Rückenplatte des eigentlichen Operationstischs auf.

Unter dem vorbestimmten Einstellbereich wird insbesondere derjenige Bereich verstanden, innerhalb dessen der Abstand zwischen dem Auflagepad und der Befestigungseinheit und somit der Abstand zwischen dem Auflagepad und der Schiene variiert werden kann.

Die Höhenverstelleinheit ist insbesondere derart ausgebildet, dass der Abstand zwischen der Befestigungseinheit und dem Auflagepad um mindestens 5 cm, vorzugsweise mindestens 8 cm, verstellt werden kann. Somit wird eine ausreichend große Verstellmöglichkeit für unterschiedliche Operationen und unterschiedlicher Anatomien der Patienten gegeben.

Der Abstand ist insbesondere stufenweise innerhalb des Einstellbereiches einstellbar. Insbesondere sind fünf gleichmäßig verteilte Stufen vorgesehen, so dass eine ausreichend kleinstufige Verstellmöglichkeit gegeben ist. Alternativ kann die Verstellung auch kontinuierlich erfolgen. Die Höhenverstelleinheit umfasst eine Verriegelungseinheit und eine Stange, wobei an einem ersten Ende der Stange das Auflagepad befestigt ist. Die Verriegelungseinheit ist relativ zur Befestigungseinheit ortsfest angeordnet und insbesondere mit dieser einstückig ausgebildet. Die Stange ist in eine Aufnahme der Verriegelungseinheit in Längsrichtung der Stange verschiebbar geführt, so dass über dieses verschiebbare Führen die Verstellbarkeit des Abstandes des fest mit der Stange verbundenen Auflagepads und der Befestigungseinheit zueinander erreicht wird.

An einer ersten Seite der Stange sind mehrere Rastaussparungen vorgesehen, die zum Einrasten eines ersten Riegels der Verriegelungseinheit dienen. Wenn der erste Riegel in einer Verriegelungsposition angeordnet ist, so greift er in eine der Rastaussparungen ein, so dass, je nach dem, in welche Rastaussparung er eingreift, ein unterschiedlicher Abstand zwischen dem Auflagepad und der Befestigungseinheit gegeben ist. Indem die Stange entsprechend weiter in die Aufnahme hinein bzw. hindurch bewegt wird, kann der Abstand verändert werden. Wenn der erste Riegel in eine der Rastaussparungen einrastet, so unterbindet er zumindest ein Bewegen der Stange in eine erste Richtung, wobei diese erste Richtung insbesondere bei der Befestigung der Patientenlagereinheit an einer horizontal ausgerichteten Schiene vertikal nach unten gerichtet ist. Somit wird über den ersten Riegel verhindert, dass, wenn dieser in der Verriegelungsposition angeordnet ist, sich die Stange und somit das Auflagepad nach unten bewegen kann. Hierdurch wird eine sichere Lagerung des Patienten erreicht.

Die ersten Rastaussparungen sind in die erste Richtung gesehen insbesondere derart abgeschrägt ausgebildet, dass die Stange auch dann, wenn der erste Riegel in der Verriegelungsposition angeordnet ist, in eine der ersten Richtung entgegengesetzte zweite Richtung, also insbesondere nach oben, bewegbar ist. Durch das abgeschrägte Ausbilden in die erste Richtung wird der Riegel bei einem Bewegen der Stange in die zweite Richtung, also auf die abgeschrägte Kante zu, automatisch aus der Verriegelungsposition bewegt, so dass die Stange in die zweite Richtung bewegt werden kann. In die zweite Richtung gesehen dagegen ist die Aussparung nicht abgeschrägt, so dass ein Bewegen in die erste Richtung der Stange unterdrückt wird. Vorzugsweise ist auch der erste Riegel derart abgeschrägt, dass er diese automatische Bewegung des ersten Riegels aus der Verriegelungsposition heraus unterstützt.

Ferner ist es vorteilhaft, wenn der erste Riegel zwischen der Verriegelungsposition und einer entriegelten Position bewegbar ist, wobei der erste Riegel in der entriegelten Position ein Bewegen der Stange in die erste Richtung nicht verhindert. Der erste Riegel ist insbesondere um eine Drehachse verschwenkbar gelagert und wird zwischen der Verriegelungsposition und der entriegelten Position um diese Drehachse gedreht.

Bei einer besonders bevorzugten Ausführungsform ist der erste Riegel mit Hilfe eines elastischen Elementes in der Verriegelungsposition vorgespannt und es ist ein erster Hebel zum Bewegen des ersten Riegels entgegen der Rückstellkraft des elastischen Elementes von der Verriegelungsposition in die entriegelte Position vorgesehen. Somit wird sichergestellt, dass der Riegel automatisch in die Rastaussparungen einrastet und dieses nicht unbeabsichtigt vergessen werden kann. Das elastische Element umfasst insbesondere eine Biegefeder, vorzugsweise eine Kunststoffbiegefeder, so dass ein sicheres Halten des Riegels in Verriegelungsposition gewährleistet ist. An einer zweiten Seite der Stange sind mehrere zweite Rastaussparungen vorgesehen. In diesem Fall umfasst auch die Verriegelungseinheit einen zweiten Riegel, wobei der zweite Riegel in einer Verriegelungsposition in eine der zweiten Rastaussparungen eingreift und somit ein Bewegen der Stange in die erste Richtung verhindert. Somit erfolgt die Verriegelung der Stange über zwei getrennt voneinander arbeitende Verriegelungsmechanismen, so dass ein besonders sicheres Halten des Auflagepads in der gewünschten Position erfolgt und insbesondere ein unbeabsichtigtes Absacken des Auflagepads vermieden wird. Insbesondere wird durch die zwei getrennten Verriegelungsmechanismen auch verhindert, dass die Verriegelung unbeabsichtigt gelöst wird und somit ein Patient gegebenenfalls unbeabsichtigt nach unten absackt. Jeder der Verriegelungsmechanismen ist so ausgelegt, dass er das zulässige Auflagegewicht der Patientenlagereinheit zuverlässig hält. Somit sind der erste Verriegelungsmechanismus und der zweite Verriegelungsmechanismus redundant ausgeführt.

Die ersten und die zweiten Rastaussparungen sind insbesondere an gegenüberliegenden Seiten der Stange angeordnet. Vorzugsweise sind der über den erste Riegel und die ersten Rastausparung gebildeter erster Verriegelungsmechanismus und über den zweiten Riegel und die zweite Rastaussparung gebildete zweite Verriegelungsmechanismus analog zueinander aufgebaut.

Auch die zweiten Rastaussparungen sind insbesondere in die erste Richtung gesehen derart abgeschrägt, dass die Stange auch dann, wenn der zweite Riegel in der Verriegelungsposition angeordnet ist, in die zweite Richtung bewegbar ist. Somit wird erreicht, dass die Stange und somit das Auflagepad, um diese nach oben zu bewegen, also den Abstand zur Befestigungseinheit zu vergrößern, einfach nur nach oben gezogen werden müssen, ohne dass hierfür die Hebel betätigt und die Riegel in die entriegelte Position bewegt werden müssen. Die Rastaussparungen und die Riegel wirken somit wie ein Freilauf entgegen der ersten Bewegungsrichtung. Vorzugsweise ist auch der zweite Riegel derart abgeschrägt, dass er eine automatische Bewegung des zweiten Riegels aus der Verriegelungsposition heraus unterstützt. Umgekehrt wird durch beide Riegel für sich jedoch ein Bewegen der Stange in die erste Richtung, also nach unten, vermieden. Hierbei sind bei Verriegelungsmechanismen derart ausgelegt, dass sowohl der erste als auch der zweite Riegel in der Lage sind, das maximal zulässige auf dem Auflagepad aufliegende Gewicht zu halten. Somit wird, auch wenn nur einer der Riegel in der Verriegelungsposition angeordnet ist, ein Bewegen der Stange in die erste Richtung immer noch sicher vermieden.

Auch der zweite Riegel ist zwischen einer Verriegelungsposition und einer entriegelten Position bewegbar, insbesondere verschwenkbar, wobei der zweite Riegel in der entriegelten Position ein Bewegen der Stange in die erste Richtung zulässt.

Ferner ist es vorteilhaft, wenn auch der zweite Riegel mit Hilfe eines elastischen Elementes, insbesondere einer Biegefeder, vorzugweise einer Kunststoffbiegefeder, in der Verriegelungsposition vorgespannt ist und wenn ein zweiter Hebel zum Bewegen des zweiten Riegels entgegen der Rückstellkraft des elastischen Elementes von der Verriegelungsposition in die entriegelte Position vorgesehen ist.

Bei einer besonders bevorzugten Ausführungsform müssen der erste Hebel und der zweite Hebel zum Entriegeln, also zum Bewegen des ersten bzw. zweiten Riegels von der Verriegelungsposition in die entriegelte Position, in entgegengesetzte Richtungen betätigt werden. Insbesondere müssen die beiden Hebel aufeinander zu bewegt werden. Hierdurch wird zum einen eine einfache Betätigung mit einer Hand erreicht, indem der eine Hebel mit dem Daumen und der andere Hebel mit dem Zeigefinger an ihrer Außenseite gefasst und aufeinander zubewegt werden. Zum anderen wird durch das Bewegen in die entgegengesetzte Richtung auch ein unbeabsichtigtes Betätigen, z.B. durch ein Vorbeistreifen, vermieden.

Die Enden der Hebel, die von der Bedienperson der Patientenlagereinheit betätigt werden, also diejenigen Enden, die den Riegeln abgewandt sind, sind insbesondere innerhalb einer Aussparung eines Gehäuses angeordnet. Auch hierdurch wird vermieden, dass die Hebel unbeabsichtigt betätigt und somit die Verriegelung der Höhenverstelleinheit versehentlich gelöst werden kann.

Die Stange ist insbesondere nur dann in die erste Richtung bewegbar, wenn sowohl der erste Riegel als auch der zweite Riegel in der entriegelten Position angeordnet sind. Hierzu sind beide Riegel derart ausgebildet, dass sie alleine das maximal zulässige Gewicht für die Patientenlagereinheit halten können. Somit wird doppelte Verriegelung erreicht, und unbeabsichtigtes Lösen vermieden.

Die Stange weist insbesondere eine Nut auf, in die ein fest mit der Befestigungseinheit verbundener Stift hineinragt. Der Stift ist hierbei innerhalb der Nut geführt. Durch die Nut wird zum einen ein Verdrehen der Stange relativ zur Befestigungseinheit, und somit auch ein Verdrehen des fest mit der Stange verbundenen Auflagepads, vermieden. Zum anderen wird durch die Nut der Einstellbereich, innerhalb dessen die Höhenverstellung möglich ist, beschränkt. Somit wird insbesondere auch vermieden, dass eine Bedienperson der Patientenlagereinheit sich die Finger zwischen dem Auflagepad und der Befestigungseinheit einklemmen kann, weil die Nut derart ausgebildet ist, dass immer ein ausreichend großer Mindestabstand zwischen der Oberseite der Befestigungseinheit und der Unterseite des Auflagepads gegeben ist.

An mindestens einem Ende der Nut ist vorzugsweise ein elastischer Anschlag vorgesehen, der zum einen dazu dient, einen Toleranzausgleich herzustellen und zum anderen zur Dämpfung der Bewegung am Ende dient.

Ferner ist es vorteilhaft, wenn das Auflagepad, die Höhenverstelleinheit und/oder die Befestigungseinheit metallfrei ausgebildet sind. Insbesondere sind die genannten Einheiten aus einem zur Aufnahme eines guten Röntgenbildes ausreichend röntgenstrahlendurchlässigen Material, vorzugsweise aus einem kohlefaserverstärkten Kunststoff ausgebildet. Hierdurch wird erreicht, dass auch ein Röntgenbild oder eine 3D-Röntgenaufnahme im Bereich der Auflagepads aufgenommen werden kann und diese sich in dem Röntgenbild nicht oder zumindest nur in einem zufriedenstellend geringen Maße in mindestens einem Flächenbereich gleichmäßig sichtbar sind. Somit werden Fehlinterpretationen des Röntgenbildes vermieden.

Die Befestigungseinheit umfasst insbesondere eine U-förmige Aufnahme, die bei der Montage der Patientenlagereinheit von oben auf die entsprechende Schiene aufgesteckt wird, so dass die Schiene innerhalb der U-förmigen Aufnahme aufgenommen ist. An einem Schenkel der U-förmigen Aufnahme ist verschwenkbar eine Verschlussplatte angeordnet, die über eine Schraube an dem anderen Schenkel befestigt werden kann, so dass sich ein geschlossener rechteckförmiger Querschnitt ergibt, innerhalb dessen die Schiene aufgenommen ist. Auf diese Weise wird eine besonders einfach lös- und wiederherstellbare Schnellbefestigung der Patientenlagereinheiten an den Schienen erreicht. Die Schraube kann insbesondere manuell ohne Werkzeug festgedreht und gelöst werden.

Bei einer besonders bevorzugten Ausführungsform ist zusätzlich zur Höhenverstelleinheit auch eine Querverstelleinheit vorgesehen, mit deren Hilfe das Auflagepad relativ zur Höhenverstelleinheit in einem vorbestimmten Querverstellbereich bewegbar ist. Die Querverstelleinheit umfasst insbesondere eine an dem Auflagepad angeordnete Schiene, in die das erste Ende der Stange eingreift. Hierzu ist das Ende komplementär zur Schienenform ausgebildet.

Durch die Querverstellung wird erreicht, dass der Abstand von Auflagepads zweier an parallel zueinander verlaufenden benachbarten Schienen benachbart zueinander angeordneter Patientenlagereinheiten verstellt werden kann, so dass der Freiraum zwischen den Auflagepads gegebenenfalls vergrößert werden kann, um somit einen größeren Röntgenbereich zu schaffen. Umgekehrt kann der Abstand verkleinert werden, wenn dies für die Lagerung des Patienten sinnvoll ist.

Die Verstellrichtungen der Höhenverstelleinheit und der Querverstelleinheit, also die beiden Richtungen, in denen das Auflagepad mit Hilfe der jeweiligen Verstelleinheit verstellt werden kann, sind insbesondere orthogonal zueinander gerichtet. Das Auflagepad ist relativ zur Stange, insbesondere in eine dritte Richtung verschiebbar, die orthogonal zur ersten und zur zweiten Richtung gerichtet ist.

Bei einer besonders bevorzugten Ausführungsform sind sowohl die erste, die zweite als auch die dritte Richtung jeweils orthogonal zur derjenigen Richtung, in der sich die Patientenlagereinheit auf der Schiene verschieben lässt gerichtet, so dass insgesamt die Patientenlagereinheit in drei Richtungen, die alle orthogonal zueinander stehen, verstellt werden kann. Somit ergibt sich eine größtmögliche individuelle Anpassbarkeit an die individuellen Gegebenheiten eines Patienten, was eine optimale Lagerung gewährleistet.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Figur 1: eine schematische, perspektivische Darstellung einer Anordnung zum Lagern eines zu röntgenden Patienten während einer Operation;
- Figur 2: eine Draufsicht auf eine Vorrichtung der Anordnung nach Figur 1;
- Figur 3: eine Seitenansicht zweier Patientenlagereinheiten der Vorrichtung nach den Figur 2;
- Figur 4: eine schematische, perspektivische Darstellung einer der Patientenlagereinheiten nach Figur 3;
- Figur 5: einen Ausschnitt der Patientenlagereinheit nach Figur 4;
- Figur 6: einen weiteren Ausschnitt der Patientenlagereinheit nach Figur 4;
- Figur 7: eine Draufsicht eines Teils der Patientenlagereinheit nach den Figuren 4 bis 6;
- Figur 8: einen weiteren Ausschnitt der Patientenlagereinheit nach den Figuren 4 bis 7;
- Figur 9: einen weiteren Ausschnitt der Patientenlagereinheit nach den Figuren 4 bis 8; und
- Figur 10: einen weiteren Ausschnitt der Patientenlagereinheit nach den Figuren 4 bis 9.

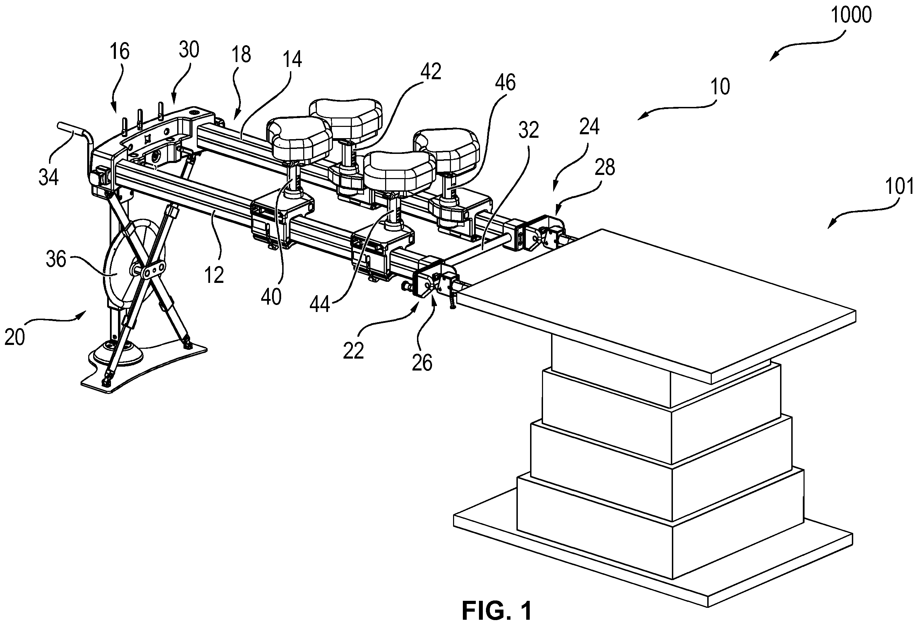

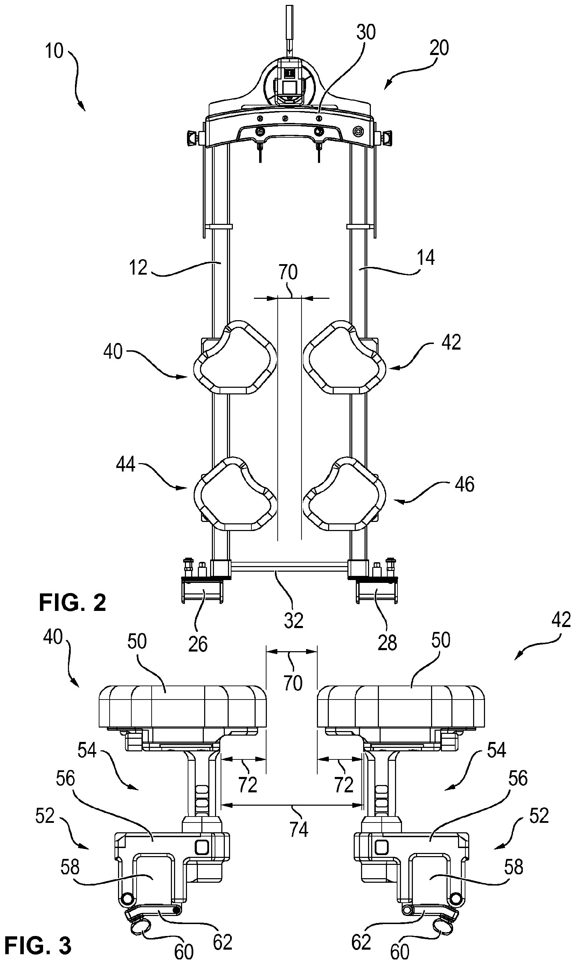

In Fig.1 ist eine schematische, perspektivisch Darstellung einer Anordnung 1000 zum Lagern eines Patienten während einer Operation dargestellt. Die Anordnung umfasst einen Operationstisch 101 sowie eine Vorrichtung 10 zum Lagern des zu röntgenden Körperbereichs des Patienten. Fig.2 zeigt eine Draufsicht auf diese Vorrichtung 10 nach Fig.1.

Die Vorrichtung 10 wird insbesondere verwendet, um Patienten während einer Rückenoperation zu lagern. Bei solchen Rückenoperationen, insbesondere an der Wirbelsäule, müssen die Patienten in der Regel während der Operation geröntgt werden, wozu ein C-förmiges Röntgengerät verwendet wird. Insbesondere wird der C-Bogen des Röntgengeräts zur Aufnahme von 3D-Bilder um den Patienten bewegt. Wie im Folgenden noch näher ausgeführt wird, ist die Vorrichtung 10 derart ausgebildet, dass mit ihr auf einfache Weise ein qualitativ hochwertiges Röntgenbild erzeugt werden kann. Ein Patient kann nicht auf einem "normalen" Operationstisch 101 gelagert werden, da ein solcher Operationstisch ein Röntgen mit der notwendigen Qualität und in den erforderlichen Bereichen nicht ermöglicht. Zum einen begrenzt die massive Fußsäule des Operationstisches 101 die mögliche Röntgenlänge und zum anderen umfasst ein normaler Operationstisch 101 zu viele metallhaltige konstruktive Elemente, die das Röntgenbild negativ beeinflussen. Außerdem ist die Patientenlagerfläche des Operationstisches 101 zu breit für die Aufnahme von 3D-Aufnahmen mit Hilfe des verwendeten C-Bogens.

Die Vorrichtung 10 umfasst zwei Schienen 12, 14, deren erste Enden 16, 18 an einem Ständer 20 gelagert sind. Die den ersten Enden 16, 18 entgegengesetzten zweiten Enden 22, 24 der Schienen 12, 14 können über Befestigungseinheiten 26, 28 an dem Operationstisch 101, insbesondere an Schnittstellen zur Verbindung des Operationstisches 101 mit Beinplatten, befestigt werden.

Der Ständer 20 dient zum einen zum Abstützen der Schienen 12, 14 auf dem Boden und zum anderen dazu, um eine vorbestimmten Abstand zwischen den Schienen 12, 14 sicherzustellen, indem der Ständer 20 eine Verbindungseinheit 30 umfasst, über die die beiden ersten Enden 16, 18 der Schienen 12, 14 miteinander verbunden sind.

Ebenso sind die zweiten Enden 22, 24 der Schienen 12, 14 über eine weitere Verbindungseinheit 32 miteinander verbunden, so dass der gewünschte Abstand zwischen den Schienen 12, 14 eingehalten ist. Die Schienen 12, 14 verlaufen parallel zueinander.

Der Ständer 20 ist derart ausgebildet, dass er höhenverstellbar ist, d.h. dass der Abstand der Schienen 12, 14 zum Boden verändert werden kann. Hierzu umfasst der Ständer 20 zum einen eine Handkurbel 34 mit deren Hilfe die Höhe verändert werden kann und zum anderen ein Handrad 36 zum Feststellen und Versteifen des Standfußes des Ständers 20.

Der Operationstisch 101, an dem die Schienen 12, 14 über die Befestigungseinheiten 26, 28 befestigt sind, ist vorzugsweise mit Hilfe eines Stellantriebs höhenverstellbar, so dass durch entsprechendes Einstellen des Ständers 20 und des Operationstisches 101 die Schienen 12, 14 in einer geeigneten Höhe horizontal angeordnet werden können.

Auf den Schienen 12, 14 sind jeweils zwei Patientenlagereinheiten 40 bis 46 zum Lagern des Patienten angeordnet. In Fig.3 sind zwei dieser Patientenlagereinheiten 40, 42 in einer Seitenansicht dargestellt, wobei zur Vereinfachung der Darstellung die Schienen 12, 14 nicht dargestellt wurden.

Die Patientenlagereinheiten 40 bis 46 umfassen jeweils ein Auflagepad 50, auf dem der Patient aufliegt. Ferner haben die Patientenlagereinheiten 40 bis 46 jeweils eine Befestigungseinheit 52 zur Befestigung der jeweiligen Patientenlagereinheit 40 bis 46 an der jeweiligen Schiene 12, 14 sowie eine Höhenverstelleinheit 54, über die das Auflagepad 50 mit der Befestigungseinheit 52 verbunden ist und über die der Abstand zwischen dem Auflagepad 50 und der Befestigungseinheit 52 eingestellt werden kann.

Die Befestigungseinheit 52 umfasst einen U-förmigen Grundkörper 56, in dessen Aussparung 58 die jeweilige Schiene 12, 14 aufgenommen werden kann. An dem offenen Ende des U-förmigen Grundkörpers ist eine über eine Schraube 60 verriegelbare Platte 62 vorgesehen. Zur Montage der Patientenlagereinheit 40 bis 46 wird die Befestigungseinheit 52 bei geöffneter Platte 62 auf die jeweilige Schiene 12, 14 aufgesetzt, so dass diese in der U-förmigen Aussparung 58 aufgenommen ist. Anschließend wird der Riegel 62 verschlossen und über die Schraube 60 gesichert, so dass eine sichere und dennoch einfache und schnelle Befestigung der Patientenlagereinheit 40 bis 46 an den Schienen 12, 14 möglich ist. Die Schraube 60 lässt sich hierzu ohne ein Werkzeug anziehen und lösen.

Die Patientenlagereinheiten 40, 42 dienen insbesondere zur Lagerung des Oberkörpers eines Patienten, wohingegen die Patientenlagereinheiten 44, 46 zur Lagerung der Hüfte des Patienten dienen. Der Patient liegt somit mit seinem Kopf in Richtung des Ständers 20, wobei seine Beine auf einem Teil der Patientenlagerfläche des eigentlichen Operationstisches 101 aufliegen. Zur Stützung des Kopfes sind insbesondere noch weitere, nicht dargestellte Patientenlagereinheiten an den Schienen 12, 14 befestigt.

Die Patientenlagereinheiten 40, 44 sind ausschließlich an der ersten Schiene 12, die Patientenlagereinheiten 42, 46 ausschließlich an der zweiten Schiene 14 befestigt. Es bestehen keine Verbindung zwischen den nebeneinander angeordneten Patientenlagereinheiten 40 und 42 sowie 44 und 46.

Die Patientenlagereinheiten 40 bis 46 sind daher unabhängig voneinander einzeln auf den Schienen 12, 14 verschiebbar, so dass zusammen mit der Höhenverstellung der Patientenlagereinheiten 40 bis 46 eine optimale Anpassung an die individuelle Anatomie eines zu operierenden Patienten möglich ist. Insbesondere müssen somit die Patientenlagereinheiten 40 bis 46, anders als dargestellt, nicht immer direkt nebeneinander angeordnet sein. Darüber hinaus können die Auflagepads 50 in unterschiedlichen Höhen angeordnet sein. Ferner kann der Abstand zwischen den Auflagepads 50 von benachbarten Patientenlagereinheiten 40 und 42 bzw. 44 und 46 variiert werden, indem die Auflagepads 50 auf der Höhenverstelleinheit 54 entsprechend quer verschiebbar gelagert sind.

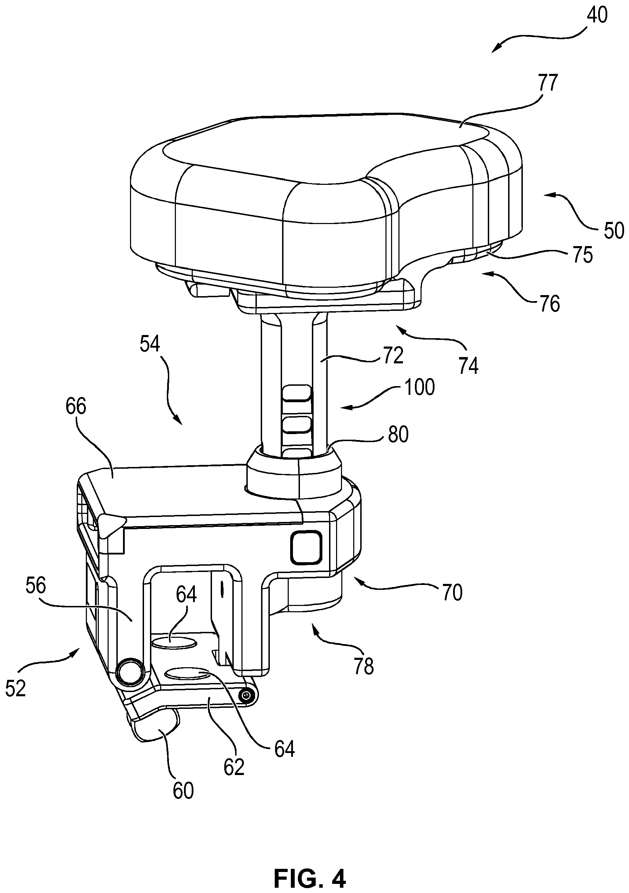

In Figur 4 ist eine schematische, perspektivische Darstellung der Patientenlagereinheit 40 gezeigt. Die andere Patientenlagereinheiten 42 bis 46 sind insbesondere analog aufgebaut, so dass der nachfolgenden Beschreibung für diese entsprechend gilt.

Auf der Innenseite der Platte 62 sind zwei Antirutschpads 64 angebracht, die einen hohen Reibungskoeffizienten aufweisen und somit ein Verrutschen der befestigten Patientenlagereinheit 40 in Längsrichtung der Schiene 12 vermeiden. Die Antirutschpads 64 sind insbesondere aus Silikon.

Die Höhenverstelleinheit 54 umfasst eine in das Gehäuse 66 der Befestigungseinheit 52 integrierte Verriegelungseinheit 70 sowie eine Stange 72, an deren ersten Ende 74 das Auflagepad 50 über eine Querverstelleinheit 76 befestigt ist. Das dem ersten Ende entgegengesetzte zweite Ende 78 der Stange 72 ist in einer Aufnahme 80 der Verriegelungseinheit 70 geführt.

Das Auflagepad 50 umfasst eine Tragestruktur 75, die insbesondere steif ausgebildet ist, und ein elastisches Polster 77, das ein bequemes Lagern des Patienten ermöglicht.

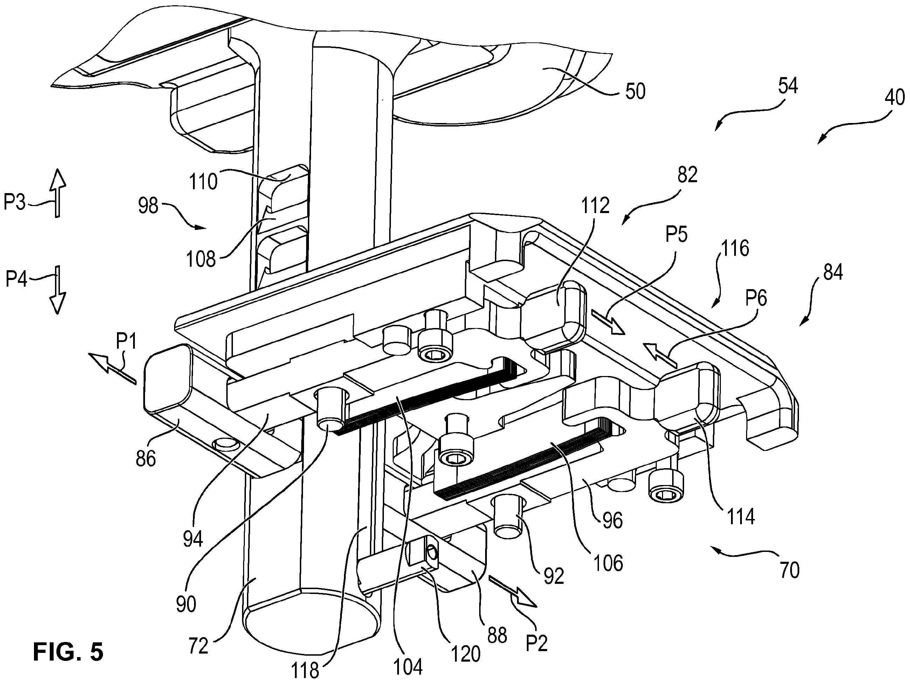

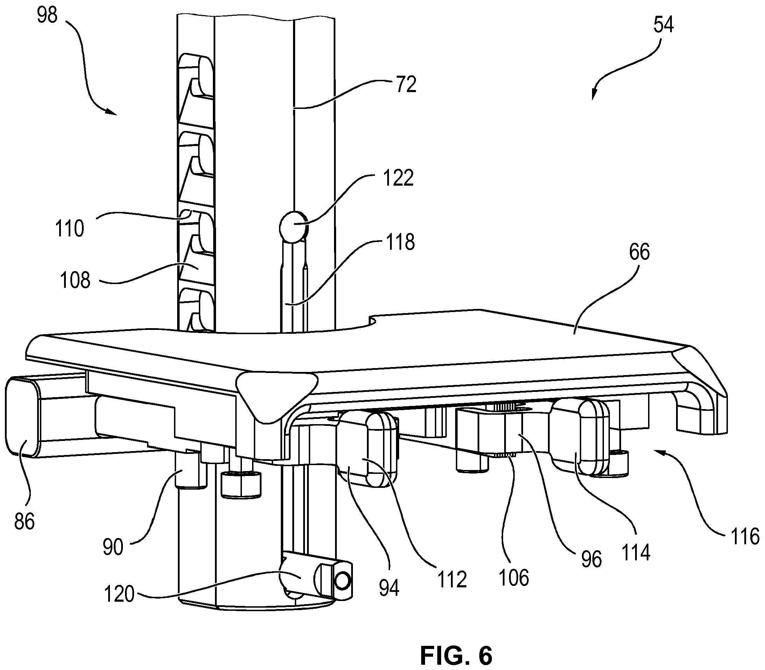

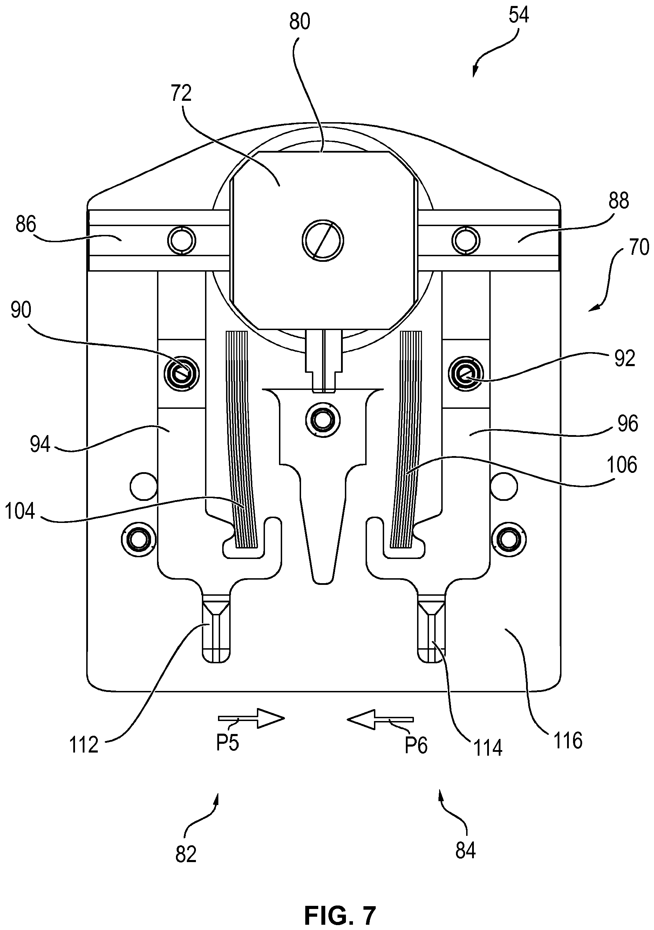

In den Figuren 5 und 6 ist jeweils eine schematische, perspektivische Darstellung der Höhenverstelleinheit 54 gezeigt, wobei ein Teil des Gehäuses 66 ausgeblendet ist, damit die innenliegenden Bauteile der Verriegelungseinheit 70 sichtbar sind. In Figur 7 ist eine Draufsicht auf die Höhenverstelleinheit 54 gezeigt, wobei zur Sichtbarkeit der Elemente der Verriegelungseinheit 70 das Gehäuse 66 sowie das gesamte Auflagepad 50 ausgeblendet sind.

Die Verriegelungseinheit 70 umfasst zwei getrennt voneinander arbeitende Verriegelungsmechanismen 82, 84. Die Verriegelungsmechanismen 82, 84 sind baugleich, vorzugsweise um eine Mittelachse der Verriegelungseinheit 70 gespiegelt, ausgebildet.

Jeder Verriegelungsmechanismus 82, 84 umfasst einen Riegel 86, 88, der eine Aussparung aufweist, in die ein um eine Drehachse 90, 92 drehbar gelagerter Hebel 94, 96 eingreift. An der Stange 72 sind an zwei gegenüberliegenden Seiten jeweils eine Vielzahl von Rastaussparungen 98, 100 ausgebildet, die komplementär zu den Riegeln 86, 88 geformt sind.

In einer Verriegelungsposition greifen die Riegel 86, 88 jeweils in eine der Rastausparungen 98, 100 ein, so dass die Stange 72 in der hierdurch vorgegebenen Position gehalten ist. Je nach dem in welche der Rastaussparungen 98, 100 die Riegel 86, 88 einrasten, ergibt sich ein anderer Abstand zwischen dem Auflagepad 50 und der Befestigungseinheit 52. Somit ist die Höhe des Auflagepads 52 relativ zu den Schienen 12, 14 einstellbar.

Jeder Verriegelungsmechanismus 82, 84 umfasst ferner eine Biegefeder 104, 106, mit deren Hilfe die Riegel 86, 88 jeweils in der verriegelten Position vorgespannt sind. Mit Hilfe der Hebel 94, 96 können die Riegel 86, 88 entgegen der Rückstellkraft der Biegefedern 104, 106 aus der Verriegelungsposition entsprechend der Pfeile P1 bzw. P2 in eine entriegelte Position verbracht werden. Werden die Hebel 84, 86 losgelassen, so werden die Riegel 86, 88 über die Rückstellkraft der entsprechenden Feder 104, 106 automatisch wieder in die Verriegelungsposition bewegt.

Die Rastaussparungen 98, 100 weisen jeweils eine abgeschrägte Kante 108 und eine nicht abgeschrägte Kante 110 auf. Durch die abgeschrägte Kante 108 wird ermöglicht, dass die Stange 72 nach oben, also in Richtung des Pfeils P3, bewegt werden kann, auch wenn die Riegel 86, 88 in der Verriegelungsposition angeordnet sind. Beim Bewegen der Stange 72 nach oben werden die Riegel 86, 88 durch die abgeschrägten Kanten 108 automatisch in die entriegelte Position bewegt, ohne dass hierfür eine Betätigung der Hebel 94, 96 notwendig ist.

Die nicht abgeschrägten Kanten 110 dagegen verhindern, dass die Stange 72 nach unten, also in Richtung des Pfeils P4 bewegt wird, es sei denn, beide Riegel 86, 88 sind in der entriegelten Position angeordnet, in der sie in keine der Rastaussparungen 98, 100 eingreifen.

Somit wird erreicht, dass zur Erhöhung des Abstandes des Auflagepads 50 zur Befestigungseinheit 52 das Auflagepad 50 und somit die Stange 72 einfach nur nach oben gezogen werden müssen, durch die doppelte Verriegelung über die beiden Verriegelungsmechanismen 82, 84 aber ein ungewolltes Bewegen des Auflagepads 50 nach unten vermieden wird. Somit wird sichergestellt, dass der Patient während der Operation in der eingestellten Höhe verbleibt.

Die Endbereiche 112, 114 der Hebel 94, 96, die zum Entriegeln betätigt werden müssen, sind insbesondere innerhalb einer Aussparung 116 des Gehäuses 66 angeordnet, so dass ein unbeabsichtigtes Betätigen der Hebel 94, 96 vermieden wird. Darüber hinaus wird unbeabsichtigtes Betätigen beider Hebel 94, 96 zusätzlich dadurch erschwert, dass die beiden Fußbereiche 112, 114 der Hebel 94, 96 in entgegengesetzten Richtungen zum Entriegeln der jeweiligen Riegel 86, 88 bewegt werden müssen. Insbesondere muss der Endbereich 112 des Hebels 94 zum Entriegeln in die Richtung P5 und der Endbereich 114 des Hebels 96 in die Richtung P6, also insbesondere aufeinander zu, bewegt werden.

Da beide Verriegelungsmechanismen 82, 84 derart ausgebildet sind, dass sie das maximal zulässige Gewicht alleine tragen können, ist somit ein Bewegen der Stange 72 nach unten, also in Richtung des Pfeils P4, nur dann möglich, wenn beide Verriegelungsmechanismen 82, 84 entriegelt wurden. Somit wird eine sehr hohe Sicherheit der Höhenverstelleinheit 54 erreicht.

Darüber hinaus ist an der Stange 72 eine Nut 118 vorgesehen, in die ein Stift 120 eingreift, der relativ zur Befestigungseinheit 52 fest an dieser angeordnet ist. Durch den in der Nut 118 geführten Stift 120 wird zum einen ein Verdrehen der Stange 72 vermieden und zum anderen wird der Einstellbereich, innerhalb dessen die Höhe verstellt werden kann, also innerhalb dessen der Abstand zwischen Auflagepad 50 und Befestigungseinheit 52 variiert werden kann, hierdurch begrenzt.

An dem dem Auflagepad 50 zugewandten Ende der Nut 118 ist insbesondere ein elastischer Anschlag 122 in der Nut 118 vorgesehen, durch den das Anschlagverhalten den Stiftes 120 an dem Nutende gedämpft und ein Toleranzausgleich hergestellt wird.

Die Höhenverstelleinheit 54 ist in dem gezeigten Ausführungsbeispiel derart ausgebildet, dass der Abstand zwischen dem Auflagepad 50 und der Befestigungseinheit 52 insgesamt um 8 cm innerhalb von fünf Stufen verstellt werden kann. Alternativ kann die Höhenverstellung auch einen größeren oder kleineren Verstellbereich aufweisen. Ebenso kann eine andere Anzahl von Stufen, beispielsweise 4 oder 6 Stufen vorgesehen sein. Dementsprechend ist eine andere Anzahl an Rastaussparungen 98, 100 vorgesehen.

Bei einer alternativen Ausführungsform der Erfindung kann auch lediglich ein Verriegelungsmechanismus 82, 84 vorgesehen sein. Alternativ können ebenfalls zwei Verriegelungsmechanismen 82, 84 vorgesehen sein, die jedoch über einen gemeinsamen Hebel betätigbar sind.

Die einzelnen Bauteile der Patientenlagereinheit 40 sind insbesondere aus einem kohlefaserverstärkten Kunststoff oder einem anderen stabilen und dennoch ausreichend röntgenstrahlendurchlässigem Material ausgebildet. Insbesondere ist die Patientenlagereinheit 40 metallfrei ausgebildet. Dies hat den Vorteil, dass von einem auf dem Patientenlagereinheit 40 bis 46 gelagerten Patienten ein qualitativ hochwertiges Röntgenbild auch in dem Bereich aufgenommen kann, in dem er auf dem Auflagepad 50 aufliegt.

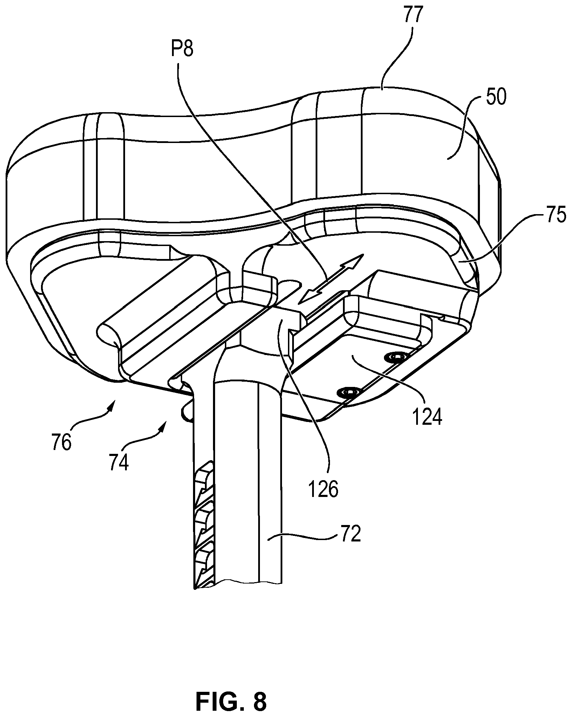

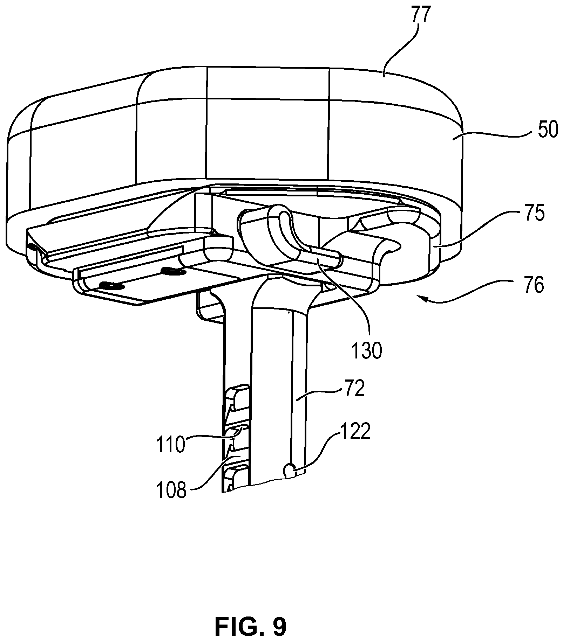

In den Figuren 8 bis 10 ist jeweils eine schematische, perspektivische Darstellung eines Ausschnitts der Patientenlagereinheit 40 nach den Figuren 4 bis 7 dargestellt, wobei insbesondere die Querverstelleinheit 76 gezeigt ist, über die das Auflagepad 50 relativ zur Stange 72 in Richtung des Doppelpfeils P8, verstellt werden kann. Hierzu ist an dem Auflagepad 50 eine Schiene 124 angeordnet, in die ein komplementär geformter Kopfbereich 126 des ersten Endes 74 der Stange 72 hineinragt. Die Schiene 124 ist insbesondere einstückig mit der Tragestruktur 75 ausgebildet.

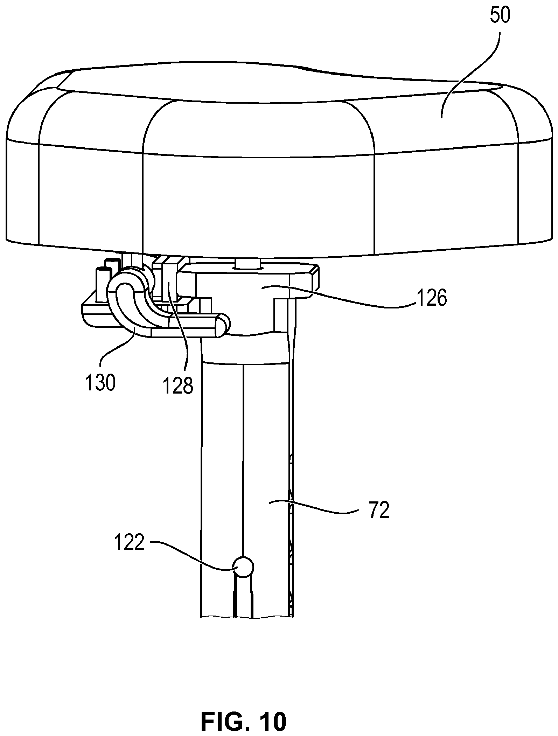

Über einen Spannhebel 130 können, wie in Figur 10 dargestellt, in der die Schiene 124 ausgeblendet ist, Spannbacken 128 gegen den Kopfbereich 126 gedrückt werden, so dass bei angezogenem Spannhebel 130 ein Verschieben des Auflagepads 50 relativ zur Stange 72 nicht möglich ist. Wird der Spannhebel 130 dagegen gelöst, so wird auch der Andruck der Spannbacken 128 gegen den Kopfbereich 126 der Stange 72 gelöst, so dass das Auflagepad 50 entsprechend in Richtung des Doppelpfeils P8 verschoben werden kann.

Über diese Querverstelleinheit 76 wird es ermöglicht, dass der Abstand zwischen den Auflagepads 50 zweier benachbarter, auf verschiedenen Schienen 12, 14 angeordneter Patientenauflageeinheiten 40, 42 bzw. 44, 46 variiert werden kann. Somit kann der Freiraum zwischen den Auflagepads 50 dieser benachbarter Patientenauflageeinheiten 40, 42 bzw. 44, 46 verändert werden, so dass die Größe eines freien Röntgenbereiches, in dem kein Material angeordnet ist, variiert werden kann.

Die Richtung P8 ist insbesondere orthogonal zu den Richtungen P3 und P4, in die die Stange 72 bewegbar ist, gerichtet. Ebenso sind die Verstellrichtung P3, P4 der Stange 72 und die Verstellrichtung P8 der Querverstelleinheit 76 orthogonal zur Längsachse der Schienen 12, 14 gerichtet, so dass eine Verstellung der Auflagepads 50 in alle drei Richtungen gegeben ist und somit die Lagerung des Patienten optimal an die individuelle Anatomie eines Patienten angepasst werden kann.

### Bezugszeichenliste

- 10: Vorrichtung
- 12, 14: Schiene
- 16, 18, 22, 24: Ende
- 20: Ständer
- 26, 28: Befestigungseinheit
- 30, 32: Verbindungseinheit
- 34: Hebel
- 36: Handrad
- 40, 42, 44, 46: Patientenlagereinheit
- 50: Auflagepad
- 52: Befestigungseinheit
- 54: Höhenverstelleinheit
- 56: Grundkörper
- 58: Aufnahme
- 60: Schraube
- 62: Platte
- 64: Antirutschpad
- 66: Gehäuse
- 70: Verriegelungseinheit
- 72: Stange
- 74: Ende
- 75: Tragestruktur
- 76: Querverstelleinheit
- 77: Polster
- 78: Ende
- 80: Aufnahme
- 82, 84: Verriegelungsmechanismus
- 86, 88: Riegel
- 90, 92: Drehachse
- 94, 96: Hebel
- 98, 100: Rastaussparung
- 101: Operationstisch
- 104, 106: Biegefeder
- 108, 110: Kante
- 112, 114: Endbereich
- 116: Aussparung
- 118: Nut
- 120: Stift
- 122: Anschlag
- 124: Schiene
- 126: Kopfbereich
- 128: Spannbacke
- 130: Spannhebel
- 1000: Anordnung
- P1 bis P8: Richtung

## Patentansprüche

1. Patientenlagereinheit für eine Vorrichtung zum Lagern eines zu röntgenden Patienten während einer Operation,
mit einer Befestigungseinheit (52) zur Befestigung der Patientenlagereinheit (40 bis 46) an einer Schiene (12 , 14) der Vorrichtung (10),
einem Auflagepad (50) zur Auflage eines Körperteils des Patienten,
und mit der Höhenverstelleinheit (54) zum Verändern des Abstandes zwischen der Befestigungseinheit (52) und dem Auflagepad (50) innerhalb eines vorbestimmten Einstellbereiches wobei die Höhenverstelleinheit (54) eine Verriegelungseinheit (70) und eine Stange (72) umfasst, und an einem ersten Ende (74) der Stange (72) das Auflagepad (50) befestigt ist, und die Verriegelungseinheit (70) relativ zur Befestigungseinheit (54) ortsfest ist, und die Stange (72) in einer Aufnahme (80) der Verriegelungseinheit (70) in Längsrichtung der Stange (72) verschiebbar geführt ist, wobei an der Stange (72) an einer ersten Seite mehrere ersten Rastaussparungen (98) vorgesehen sind, und die Verriegelungseinheit (70) einen ersten Riegel (86) umfasst, und der erste Riegel (86) in einer Verriegelungsposition wahlweise in eine der ersten Rastaussparungen (98) eingreift und ein Bewegen der Stange (72) in eine erste Richtung (P4) verhindert, **dadurch gekennzeichnet, dass** an der Stange (72) an einer zweiten Seite mehrere zweite Rastaussparungen (100) vorgesehen sind, dass die Verriegelungseinheit (70) einen zweiten Riegel (88) umfasst, und dass der zweite Riegel (88) in einer Verriegelungsposition wahlweise in eine der zweiten Rastaussparungen (100) eingreift und ein Bewegen der Stange (72) in die erste Richtung (P4) verhindert.

2. Patientenlagereinheit (40 bis 46) nach Anspruch 1, **dadurch gekennzeichnet, dass** die ersten Rastaussparungen (98) in die erste Richtung (P4) gesehen derart abgeschrägt sind, dass die Stange (72) auch dann, wenn der erste Riegel (86) in der Verriegelungsposition angeordnet ist, in eine der ersten Richtung (P4) entgegengesetzte zweite Richtung (P3) bewegbar ist.

3. Patientenlagereinheit (40 bis 46) nach einem der Ansprüche 1 oder 2, **dadurch gekennzeichnet, dass** der erste Riegel (86) zwischen der Verriegelungsposition und einer entriegelten Position bewegbar, insbesondere verschwenkbar, ist, wobei der erste Riegel (86) in der entriegelten Position ein Bewegen der Stange (72) in die erste Richtung (P4) zulässt.

4. Patientenlagereinheit (40 bis 46) nach Anspruch 3, **dadurch gekennzeichnet, dass** der erste Riegel (86) mit Hilfe eines elastischen Elementes (104), insbesondere einer Biegefeder, in der Verriegelungsposition vorgespannt ist, und dass ein erster Hebel (94) zum Bewegen des ersten Riegels (86) entgegen der Rückstellkraft des elastischen Elements (104) von der Verriegelungsposition in die entriegelte Position vorgesehen ist.

5. Patientenlagereinheit (40 bis 46) nach einem der vorhergehenden Ansprüche, **dadurch gekennzeichnet, dass** die zweiten Rastaussparungen (100) in die erste Richtung (P4) gesehen derart abgeschrägt sind, dass die Stange (72) auch dann, wenn der zweite Riegel (88) in der Verriegelungsposition angeordnet ist, in die zweite Richtung (P3) bewegbar ist.

6. Patientenlagereinheit (40 bis 46) nach einem der vorhergehenden Ansprüche, **dadurch gekennzeichnet, dass** der zweite Riegel (88) zwischen der Verriegelungsposition und einer entriegelten Position bewegbar, insbesondere verschwenkbar, ist, wobei der zweite Riegel (88) in der entriegelten Position ein Bewegen der Stange (72) in die erste Richtung (P4) zulässt.

7. Patientenlagereinheit (40 bis 46) nach Anspruch 6, **dadurch gekennzeichnet, dass** der zweite Riegel (88) mit Hilfe eines elastischen Elementes (106), insbesondere einer Biegefeder, in der Verriegelungsposition vorgespannt ist, und dass ein zweiter Hebel (96) zum Bewegen des zweiten Riegels (88) entgegen der Rückstellkraft des elastischen Elements (106) von der Verriegelungsposition in die entriegelte Position vorgesehen ist.

8. Patientenlagereinheit (40 bis 46) nach Anspruch 7, **dadurch gekennzeichnet, dass** die Verriegelungseinheit (70) derart ausgebildet ist, dass der erste Hebel (94) und der zweite Hebel (96) zum Entriegeln in entgegensetzte Richtungen (P5, P6), insbesondere aufeinander zu, bewegt werden müssen.

9. Patientenlagereinheit (40 bis 46) nach Anspruch 7 oder 8, **dadurch gekennzeichnet, dass** die manuell zu betätigenden Enden (112 , 114) der Hebel (94 , 96) in einer als Betätigungsschutz dienenden Aussparung (116) angeordnet sind.

10. Patientenlagereinheit (40 bis 46) nach einem der Ansprüche 3 bis 9, **dadurch gekennzeichnet, dass** die Stange (72) nur dann in die erste Richtung (P4) bewegbar ist, wenn sowohl der erste Riegel (86) als auch der zweite Riegel (88) in der entriegelten Position angeordnet sind.

11. Patientenlagereinheit (40 bis 46) nach einem der vorhergehenden Ansprüche, **dadurch gekennzeichnet, dass** in der Stange (72) eine Nut (118) vorgesehen ist, in die ein fest mit der Befestigungseinheit (52) verbundener Stift (120) hineinragt.

12. Patientenlagereinheit (40 bis 46) nach Anspruch 11, **dadurch gekennzeichnet, dass** an mindestens einem Ende der Nut (118) in dieser ein elastischer Anschlag (122) vorgesehen ist.

13. Patientenlagereinheit (40 bis 46) nach einem der vorhergehenden Ansprüche, **dadurch gekennzeichnet, dass** das Auflagepad (50), die Höhenverstelleinheit (54) und/oder die Befestigungseinheit (52) metallfrei, insbesondere aus einem röntgenstrahlendurchlässigen Material, vorzugsweise aus einem kohlefaserverstärkten Kunststoff, ausgebildet sind.

14. Patientenlagereinheit (40 bis 46) nach einem vorhergehenden Ansprüche, **dadurch gekennzeichnet, dass** die Befestigungseinheit (52) eine U-förmige Aufnahme (56) zur Aufnahme der Schiene (12, 14) und eine Platte (62) zum Verschließen der Öffnung der U-förmigen Aufnahme (56) nach der Montage umfasst.

15. Patientenlagereinheit (40 bis 46) nach einem der vorhergehenden Ansprüche, **dadurch gekennzeichnet, dass** eine Querverstelleinheit (76) vorgesehen ist, mit deren Hilfe das Auflagepad (50) relativ zur Höhenverstelleinheit (54) in einem vorbestimmten Querverstellbereich bewegbar ist, wobei insbesondere die Verstellrichtungen der Höhenverstelleinheit (54) und der Querverstelleinheit (76) orthogonal zueinander gerichtet sind.

## Claims

1. A patient-supporting unit for a device for supporting a patient who is to be X-rayed during an operation,
comprising a fastening unit (52) for fastening the patient-supporting unit (40 through 46) to a rail (12, 14) of the device (10),
a resting pad (50) for resting a body part of the patient thereon,
and a height adjustment unit (54) for changing the distance between the fastening unit (52) and the resting pad (50) within a predetermined setting range,
wherein the height adjustment unit (54) includes a locking unit (70) and a rod (72), and the resting pad (50) is fastened to a first end (74) of the rod (72), and the locking unit (70) is stationary relative to the fastening unit (54), and the rod (72) is slidably guided in a receptacle (80) of the locking unit (70) in the longitudinal direction of the rod (72),
wherein multiple first snap-in recesses (98) are provided on a first side of the rod (72), and the locking unit (70) includes a first locking bar (86), and the first locking bar (86) in a locked position selectively engages in one of the first snap-in recesses (98) and prevents movement of the rod (72) in a first direction (P4), **characterized in that**
multiple second snap-in recesses (100) are provided on a second side of the rod (72), the locking unit (70) includes a second locking bar (88), and the second locking bar (88) in a locked position selectively engages in one of the second snap-in recesses (100) and prevents movement of the rod (72) in the first direction (P4).

2. The patient-supporting unit (40 through 46) according to Claim 1, **characterized in that** the first snap-in recesses (98), viewed in the first direction (P4), are beveled in such a way that the rod (72) is movable in a second direction (P3) opposite the first direction (P4), even when the first locking bar (86) is in the locked position.

3. The patient-supporting unit (40 through 46) according to one of Claims 1 or 2, **characterized in that** the first locking bar (86) is movable, in particular pivotable, between the locked position and an unlocked position, wherein the first locking bar (86) in the unlocked position allows movement of the rod (72) in the first direction (P4).

4. The patient-supporting unit (40 through 46) according to Claim 3, **characterized in that** the first locking bar (86) is pretensioned in the locked position with the aid of an elastic element (104), in particular a flexible spring, and a first lever (94) is provided for moving the first locking bar (86) from the locked position into the unlocked position, against the restoring force of the elastic element (104).

5. The patient-supporting unit (40 through 46) according to one of the preceding claims, **characterized in that** the second snap-in recesses (100), viewed in the first direction (P4), are beveled in such a way that the rod (72) is movable in the second direction (P3), even when the second locking bar (88) is in the locked position.

6. The patient-supporting unit (40 through 46) according to one of the preceding claims, **characterized in that** the second locking bar (88) is movable, in particular pivotable, between the locked position and an unlocked position, wherein the second locking bar (88) in the unlocked position allows movement of the rod (72) in the first direction (P4).

7. The patient-supporting unit (40 through 46) according to Claim 6, **characterized in that** the second locking bar (88) is pretensioned in the locked position with the aid of an elastic element (106), in particular a flexible spring, and a second lever (96) is provided for moving the second locking bar (88) from the locked position into the unlocked position, against the restoring force of the elastic element (106).

8. The patient-supporting unit (40 through 46) according to Claim 7, **characterized in that** the locking unit (70) is designed in such a way that for unlocking, the first lever (94) and the second lever (96) must be moved in opposite directions (P5, P6), in particular toward one another.

9. The patient-supporting unit (40 through 46) according to Claim 7 or 8, **characterized in that** the ends (112, 114) of the levers (94, 96) to be manually actuated are situated in a recess (116) that serves as actuation protection.

10. The patient-supporting unit (40 through 46) according to one of Claims 3 to 9, **characterized in that** the rod (72) is movable in the first direction (P4) only when both the first locking bar (86) and the second locking bar (88) are in the unlocked position.

11. The patient-supporting unit (40 through 46) according to one of the preceding claims, **characterized in that** provided in the rod (72) is a groove (118) into which a pin (120) that is fixedly connected to the fastening unit (52) protrudes.

12. The patient-supporting unit (40 through 46) according to Claim 11, **characterized in that** an elastic stop (122) is provided in the groove (118) at at least one end thereof.

13. The patient-supporting unit (40 through 46) according to one of the preceding claims, **characterized in that** the resting pad (50), the height adjustment unit (54), and/or the fastening unit (52) are/is metal-free, in particular made of a material that is permeable to X-rays, preferably a carbon fiber-reinforced plastic.

14. The patient-supporting unit (40 through 46) according to one [of the] preceding claims, **characterized in that** the fastening unit (52) includes a U-shaped receptacle (56) for accommodating the rail (12, 14), and a plate (62) for closing the opening in the U-shaped receptacle (56) after installation.

15. The patient-supporting unit (40 through 46) according to one of the preceding claims, **characterized in that** a cross adjustment unit (76) is provided, by means of which the resting pad (50) is movable relative to the height adjustment unit (54) in a predetermined cross adjustment range, wherein in particular the adjustment directions of the height adjustment unit (54) and of the cross adjustment unit (76) are oriented orthogonally with respect to one another.

## Revendications

1. Unité de support pour patient pour un dispositif permettant de coucher un patient à radiographier pendant une opération, comprenant
une unité de fixation (52) pour fixer l'unité de support pour patient (40 à 46) à un rail (12, 14) du dispositif (10),
un élément de soutien (50) pour soutenir une partie du corps du patient, et
une unité de réglage en hauteur (54) pour modifier la distance entre l'unité de fixation (52) et l'élément de soutien (50) dans une plage de réglage prédéterminée,
dans laquelle l'unité de réglage en hauteur (54) comprend une unité de verrouillage (70) et une barre (72), et l'élément de soutien (50) est fixé à une première extrémité (74) de la barre (72), et l'unité de verrouillage (70) est stationnaire par rapport à l'unité de fixation (54), et la barre (72) est guidée dans un logement (80) de l'unité de verrouillage (70) de manière mobile dans le sens longitudinal de la barre (72),
dans laquelle plusieurs premiers évidements d'arrêt (98) sont prévus sur la barre (72) sur un premier côté, et l'unité de verrouillage (70) comprend un premier verrou (86), et le premier verrou (86) dans une position de verrouillage vient en prise au choix avec l'un des premiers évidements d'arrêt (98), et empêche un déplacement de la barre (72) dans une première direction (P4),
**caractérisée en ce que** sur la barre (72), sur un deuxième côté, plusieurs deuxièmes évidements d'arrêt (100) sont prévus, **en ce que** l'unité de verrouillage (70) comprend un deuxième verrou (88), et **en ce que** le deuxième verrou (88), dans une position de verrouillage, vient en prise au choix avec l'un des deuxièmes évidements d'arrêt (100) et empêche un déplacement de la barre (72) dans la première direction (P4).

2. Unité de support pour patient (40 à 46) selon la revendication 1, **caractérisée en ce que** les premiers évidements d'arrêt (98), vus dans la première direction (P4), sont biseautés de telle sorte que la barre (72) peut être déplacée dans une deuxième direction (P3) opposée à la première direction (P4) même lorsque le premier verrou (86) est disposé dans la position de verrouillage.

3. Unité de support pour patient (40 à 46) selon l'une quelconque des revendications 1 ou 2, **caractérisée en ce que** le premier verrou (86) peut être déplacé, en particulier amené à pivoter, entre la position de verrouillage et une position déverrouillée, le premier verrou (86) dans la position déverrouillée permettant un déplacement de la barre (72) dans la première direction (P4).

4. Unité de support pour patient (40 à 46) selon la revendication 3, **caractérisée en ce que** le premier verrou (86) est précontraint à l'aide d'un élément élastique (104), en particulier d'un ressort de flexion, dans la position de verrouillage, et **en ce qu'**un premier levier (94) est prévu pour déplacer le premier verrou (86) contre la force de rappel de l'élément élastique (104) de la position de verrouillage à la position déverrouillée.

5. Unité de support pour patient (40 à 46) selon l'une quelconque des revendications précédentes, **caractérisée en ce que** les deuxièmes évidements d'arrêt (100), vus dans la première direction (P4), sont biseautés de telle sorte que la barre (72) peut être déplacée dans la deuxième direction (P3) même lorsque le deuxième verrou (88) est disposé dans la position de verrouillage.

6. Unité de support pour patient (40 à 46) selon l'une quelconque des revendications précédentes, **caractérisée en ce que** le deuxième verrou (88) peut être déplacé, en particulier amené à pivoter, entre la position de verrouillage et une position déverrouillée, le deuxième verrou (88) dans la position déverrouillée permettant un déplacement de la barre (72) dans la première direction (P4).

7. Unité de support pour patient (40 à 46) selon la revendication 6, **caractérisée en ce que** le deuxième verrou (88) est précontraint à l'aide d'un élément élastique (106), en particulier d'un ressort de flexion, dans la position de verrouillage, et **en ce qu'**un deuxième levier (96) est prévu pour déplacer le deuxième verrou (88) contre la force de rappel de l'élément élastique (106) de la position de verrouillage à la position déverrouillée.

8. Unité de support pour patient (40 à 46) selon la revendication 7, **caractérisée en ce que** l'unité de verrouillage (70) est réalisée de telle sorte que pour le déverrouillage, le premier levier (94) et le deuxième levier (96) sont à déplacer dans des directions opposées (P5, P6), en particulier l'un vers l'autre.

9. Unité de support pour patient (40 à 46) selon la revendication 7 ou 8, **caractérisée en ce que** les extrémités (112, 114) à actionnement manuel des leviers (94, 96) sont disposées dans un évidement (116) servant de protection d'actionnement.

10. Unité de support pour patient (40 à 46) selon l'une quelconque des revendications 3 à 9, **caractérisée en ce que** la barre (72) ne peut être déplacée dans la première direction (P4) que si aussi bien le premier verrou (86) que le deuxième verrou (88) sont disposés dans la position déverrouillée.

11. Unité de support pour patient (40 à 46) selon l'une quelconque des revendications précédentes, **caractérisée en ce que** dans la barre (72) est prévue une rainure (118) dans laquelle fait saillie une cheville (120) reliée solidement à l'unité de fixation (52).

12. Unité de support pour patient (40 à 46) selon la revendication 11, **caractérisée en ce qu'**à au moins une extrémité de la rainure (118), une butée élastique (122) est prévue dans celle-ci.

13. Unité de support pour patient (40 à 46) selon l'une quelconque des revendications précédentes, **caractérisée en ce que** l'élément de soutien (50), l'unité de réglage en hauteur (54) et/ou l'unité de fixation (52) sont réalisés sans métal, en particulier dans un matériau transparent aux rayons X, de préférence une matière plastique renforcée en fibres de carbone.

14. Unité de support pour patient (40 à 46) selon l'une quelconque des revendications précédentes, **caractérisée en ce que** l'unité de fixation (52) comprend un logement (56) en forme de U pour recevoir le rail (12, 14) et une plaque (62) pour fermer l'ouverture du logement (56) en forme de U après le montage.

15. Unité de support pour patient (40 à 46) selon l'une quelconque des revendications précédentes, **caractérisée en ce qu'**une unité de réglage transversal (76) est prévue à l'aide de laquelle l'élément de soutien (50) peut être déplacé par rapport à l'unité de réglage en hauteur (54) dans une plage de réglage transversal prédéterminée, les directions de réglage de l'unité de réglage en hauteur (54) et de l'unité de réglage transversal (76) étant en particulier orientées perpendiculairement l'une à l'autre.
